Europäisches Patentamt

(19) European Patent Office

Office européen des brevets

(11) Publication number: **0 033 582**
**B1**

## EUROPEAN PATENT SPECIFICATION

(45) Date of publication of patent specification: **11.04.84**

(51) Int. Cl.³: **C 07 G 7/00 //A61K35/16**

(21) Application number: **81300069.2**

(22) Date of filing: **08.01.81**

(54) Method of obtaining factor VIII.

(30) Priority: **18.01.80 CA 344000**
**26.11.80 US 210385**

(43) Date of publication of application:
**12.08.81 Bulletin 81/32**

(45) Publication of the grant of the patent:
**11.04.84 Bulletin 84/15**

(84) Designated Contracting States:
**AT BE CH DE FR GB LI NL SE**

(56) References cited:
**EP - A - 0 011 231**
**GB - A - 1 178 958**
**US - A - 3 652 530**
**US - A - 4 104 266**
**US - A - 4 203 891**
**US - A - 4 210 580**
**US - E - 29 698**

**CHEMICAL ABSTRACTS, vol. 86, réf. 86854s
(1977) no. 13, 28-03-1977 page 309 Columbus,
Ohio, US S.J. SLICHTER et al.: "Preparation of
cryoprecipitated factor VIII concentrates"**

(73) Proprietor: **THE CANADIAN RED CROSS SOCIETY**
**95 Wellesley Street East**
**Toronto Ontario M4Y 1H6 (CA)**

(73) Proprietor: **Rock, Gail Ann**
**270 Sandridge Road**
**Rockcliffe Park Ottawa Ontario K1L 5A2 (CA)**

(73) Proprietor: **Palmer, Douglas Stephen**
**17 Front Street**
**Hull Quebec J8Y 3M4 (CA)**

(72) Inventor: **Rock, Gail Ann**
**270 Sandridge Road**
**Rockcliffe Park Ontario K1L 5A2 (CA)**
Inventor: **Palmer, Douglas Stephen**
**17 Front Street**
**Hull Quebec J8Y 3M4 (CA)**

(74) Representative: **Lawrence, Peter Robin Broughton
et al,
GILL JENNINGS & EVERY 53-64 Chancery Lane
London WC2A 1HN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# O 033 582

## Method of obtaining factor VIII

This invention is concerned with a method of obtaining Factor VIII without the use of chemical precipitating agents. The technique relies on the heparin-induced coprecipitation of Factor VIII with a plasma protein called cold-insoluble globulin (Clg).

In 1954, Thomas et al in a paper entitled "Cold precipitation by heparin of a protein in rabbit and human plasma" Proc. Soc. Exp. Biol. Med. 86: 813—819 reported the occurrence of heparin-induced cold or cryoprecipitate plasma from endotoxin-treated rabbits. This fraction has since come to be known as the heparin-precipitable fraction of plasma. Subsequent studies reported in 1957 by Smith and Von Korff in a paper entitled "A heparin-precipitable fraction of human plasma. I. Isolation and characterization of the fraction." J. Clin. Invest. 36: 596—604 and Smith in a paper entitled "A heparin-precipitable fraction of human plasma II. Occurrence and significance of the fraction in normal individuals and in various disease states." J. Clin. Invest., 36: 605—616, showed that large amounts of this cold-precipitable fraction were formed from plasmas of patients with certain inflammatory infectious and neo-plastic diseases when heparin was added to the plasma. In normal donors, the amount produced was usually less. At this time it was established that fibrinogen was a major component of this fraction. A second minor component of the fraction was also observed which was termed "cold-insoluble globulin". This cold-insoluble globulin (Clg) is known to be a normal plasma glycoprotein that is structurally and immunochemically distinct from fibrinogen and all other plasma proteins. Clg normally circulates at plasma levels of $0.33 \pm 0.1$ g/litre. Cold-insoluble globulin in its various forms has been designated by a variety of terms, of which "fibronectin" has recently become most accepted (The structure and Biologic Activities of Plasma Fibronectin, Mosesson et al, Blood, Volume 56, No. 2, pages 145—158).

In 1974, U.S. Patent No. 3,803,115 was issued to Fekete et al for a method of improving the yield of Factor VIII or A.H.F. obtained from blood plasma or plasma fractions which involved the addition of heparin to a concentrate of A.H.F. obtained from the plasma or plasma fraction by cryoprecipitation. This patent was reissued July 11, 1978 under Re 29698. In the reissue patent Fekete et al indicate that the addition of heparin is made to an A.H.F. concentrate obtained from blood plasma or a plasma fraction by cryoprecipitation which may then be further fractionated to obtain an even more concentrated form of A.H.F. In the reissue patent, column 2, lines 36 et al, it is indicated that the heparin is preferably added to the A.H.F.-rich concentrate in addition to citrate. A preferred method of conducting the double anticoagulant addition is to add the heparin to the cryoprecipitate in the form of a heparinized, citrated-saline solution. When the A.H.F.-rich cryoprecipitate is further fractionated to obtain a high purity, more concentrated form of A.H.F., heparin is preferably added twice, once to the initial cryoprecipitate and subsequently to the further fractionated A.H.F. concentrate. In each case, heparin is preferably added along with citrate as a second anticoagulant.

It is especially important to note that, in the original Fekete patent and the reissue patent, the plasma is received frozen from a donor centre and then a cryoprecipitate step is performed before the heparin is added. Heparin is added only to the cryoprecipitate or to the concentrate of A.H.F. after cryoprecipitation occurs. Reference should also be made to Col. 4 of the Fekete reissue patent, lines 46—49, wherein it is clear, from the temperatures at which the cryoprecipitate is resolubilized, that the Clg or fibronectin is lost and therefore unavailable to aid in the recovery of Factor VIII. According to Fekete, the yield of Factor VIII is improved from 13.6 to 12.5% in the non-heparinized procedure to 17% in the heparinized procedure. Fekete indicates that this increase of approximately 4% represents a 25% increase over the non-heparinized procedure. However, as the Table found in column 5 of the patent, Example 1, would indicate, the total yield from the cryoprecipitate is as low as 35%, representing only 350 units per litre of starting plasma. Therefore, the 17% recovery in the final product represents only 170 units per litre of starting plasma. These calculations are based on the international reference standard of one unit per ml of plasma.

It will be clear from the above discussion that the cold precipitation of fibronectin and fibrinogen had been an established procedure for many years prior to the filing date of the original Fekete patent in 1972. It was generally known that addition of heparin or some other polysaccharide compound was necessary in order to effect precipitation of fibronectin in the cold. However, at no point has the literature suggested or contained directions for the application of a cold-insoluble globulin or fibronectin step towards the production of Factor VIII. In fact, a 1975 paper by Mosher, entitled "Cross Linking of Cold-Insoluble Globulin by Fibrin-Stabilizing Factor," published in J.B.C. 250: 6614, 1975, indicated that cold-insoluble globulin was distinguished from antihemophilic factor (Factor VIII) by amino acid analysis and by the position of elution from 4% agarose gels and by the electrophoretic migration in polyacrylamide gels.

Other publications dealing with extracts of Factor VIII have occurred recently, including US Patent Specification 4,203,891 and European Patent Publications 0011231 and 0022052, each of which was published after 18th January 1980. Like the other literature discussed above, they do not suggest the particular features of the present invention.

This invention is based, at least in part, on the discovery that if heparin is present in plasma that is frozen and if cryoprecipitate obtained from that plasma is resolubilised, buffered with a buffer including heparin and is then incubated, the incubation results in the formation of a precipitate that is rich in Factor VIII so that desirable yields of Factor VIII can then be obtained from this precipitate.

In particular the method of obtaining Factor VIII according to the invention comprises freezing plasma that is freshly obtained blood plasma collected into a calcium chelating agent or that is obtained by plasmapheresis using a calcium chelating agent, resolubilising the frozen plasma, resolubilising a cryoprecipitate obtained from the resolubilised plasma, adding a citrate saline buffer to the resolubilised cryoprecipitate and separting Factor VIII from the resolubilised cryoprecipitate, and this method is characterized in that heparin is incorporated with the plasma before it is frozen, the buffer includes heparin and the separation of the Factor VIII from the resolubilised cryoprecipitate is effected by incubating the resolubilised cryoprecipitate in the presence of cold insoluble globulin that is precipitatable by heparin and thereby obtaining a precipitate rich in Factor VIII and also including cold insoluble globulin, and isolating Factor VIII from this precipitate.

Generally the plasma that is to be frozen is freshly obtained blood plasma that has been collected into a calcium chelating anticoagulant, preferably a citrate phosphate dextrose anticoagulant, and to which heparin is added or is blood plasma obtained by plasmapheresis using heparin and a calcium chelating anticoagulant, generally sodium citrate.

The plasma is frozen, typically at about −80°C. It may then be resolubilised generally at a temperature of from 0°C to 10°C for 30 to 120 minutes, preferably at about 4°C for about 75 minutes.

A cryoprecipitate may be isolated from the resolubilised plasma by centrifuging and may be resolubilised at a temperature of about 37°C for 2 to 5 minutes. The resolubilisation may be conducted in a conventional blood transfusion bag and the amount of citrate saline heparin buffer added to each resolubilised cryoprecipitate bag may be from 2 to 10 ml.

The buffered, resolubilised cryoprecipitate is incubated under conditions, such as a temperature of from 0 to 10°C for a time of at least 1 hour, such that Factor VIII present in the cryoprecipitate is insolubilised using heparin-precipitatable cold-insoluble globulin so as to form a Factor VIII-rich precipitate. Generally the incubation is conducted at about 0°C for about 2 hours.

The calcium chelating anticoagulant can be selected from any of the well known anticoagulants which function by lowering the physiological level of calcium in the blood or blood plasma. The preferred such anticoagulants are the citrate anticoagulants, ethylenediaminetetraacetic acid (EDTA) and oxalates. Of these, the citrate anticoagulants are preferred. The preferred citrate anticoagulants include acid-citrate-dextrose (ACD) and citrate-phosphate-dextrose (CPD).

The essential steps of the method of this invention are the addition of the citrate saline heparin (CSH) buffer to the resolubilised cryoprecipitate and the incubation of the cryoprecipitate.

The buffer is preferably added in amounts ranging from about 2 to about 4 ml per cryoprecipitate bag and, most preferably, an addition of 2.5 ml per cryoprecipitate bag is made. The buffer preferably has a composition of 0.2 M sodium citrate, 0.9% by weight of sodium chloride and 1 unit per ml of buffer of sodium heparin. The pH of the buffer is maintained at 7.2.

Cold insoluble globulin (CIg) is not insolubilised after treatment of CPD plasma with heparin. The precipitate of the CIg is not formed until a specific set of conditions is met. Thus, while these conditions include introduction of heparin to fresh CPD plasma, this is not sufficient in itself to produce CIg. Indeed, visual observation indicated that this step alone does not produce the CIg since, when present, this material is seen as a white deposit at the bottom of a blood bag. These steps are the addition of the CSH buffer to the cryoprecipitate and the cold incubation step which permits formation of the precipitating globulin. If these combined steps are not employed, cold-insoluble globulin is not formed even in the presence of heparin.

Factor VIII may be isolated from the Factor VIII-rich precipitate in the cold at a temperature of from 0°C to 10°C, preferably at about 0°C, by washing with citrate saline heparin buffer. The precipitate is then drained and resolubilised with further citrate saline heparin buffer, preferably at about 37°C for 2 to 5 min. The Factor VIII-rich solution can then be separated from the remaining debris, most easily by centrifugation. The Factor VIII-rich solution obtained will be of intermediate purity when the starting plasma is obtained from whole blood from which the red blood cells have been separated. In the case where the starting plasma is pheresis plasma, the Factor VIII will have an activity which qualifies it as high purity.

Alternatively, or when required, the Factor VIII-rich precipitate can be subjected to conventional processes for obtaining high purity Factor VIII, examples of which are fractionation by precipitation, by glycine, ethanol, ethanol-glycine, polyethylene glycol, or glycine-polyethylene glycol precipitation and/or other known purification agents.

The heparin is preferably added at a concentration of 1 unit heparin per ml of plasma, although a range of from about 1 to about 10 units heparin per ml of plasma is effective. Sodium heparin has proved effective in this method.

The method in precise terms involves freezing the fresh CPD plasma with the heparin added thereto at −80°C and then resolubilizing at 4°C for 75 minutes, after which it is centrifuged (spin 7000 × g, 7 min. at 0°C). The resulting cryoprecipitate is allowed to resolubilize at 37°C for 2—5 minutes, after which 2.5 ml of CSH (citrate saline heparin) buffer is added per cryoprecipitate bag. The resolubilized, pooled cryoprecipitate is then incubated at 0°C for two hours in a refrigerated water bath.

The Factor VIII present in the cryoprecipitate pool now insolubilizes along with the cold-insoluble globulin, and the total insoluble precipitate is then separated from the Factor VIII-poor supernatant by centrifugation (spin 7000 × g, 7 min. at 0°C). After draining off the supernatant, the cold-insoluble precipitate is washed at 0°C with 10 ml of cold CSH buffer, drained and allowed to resolubilize at 37°C for five minutes with from 2—10 ml, preferably two ml, of CSH buffer per original cryoprecipitate bag. A further centrifugation (spin 3700 × g, 6 min.) is conducted and the Factor VIII-rich solution is separated from the debris.

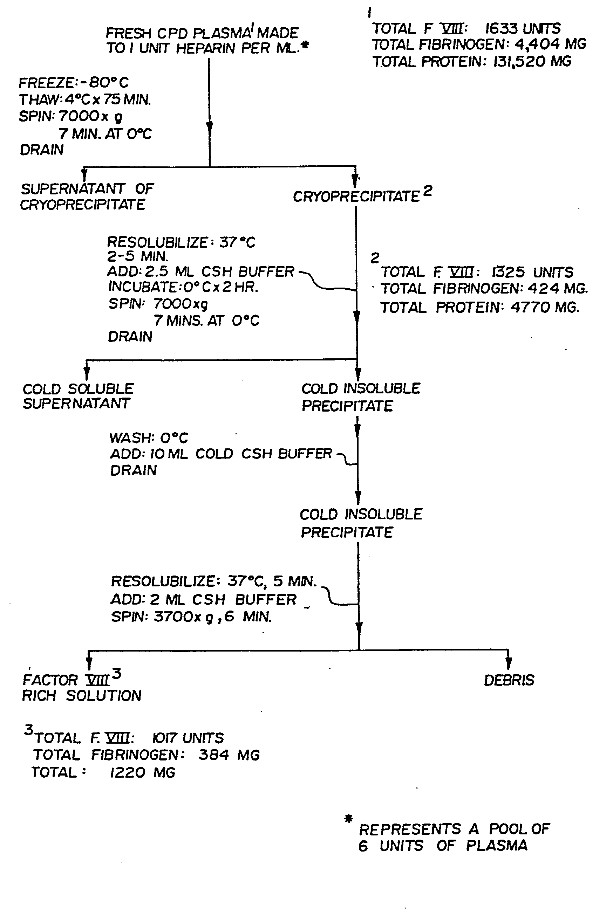

The accompanying drawing is a chart illustrating the invention, the particular steps being illustrated for a pool of six units of plasma. The previous specifically described method is illustrated by this drawing.

In the following Table 1, there is provided an analysis of the given illustrative embodiment of the invention. CPD plasma was separated from red blood cells (RBC) and made to one unit with sodium heparin, per ml of freshly-obtained human blood blasma.

The plasma was frozen (—80°C) and stored (—60°C) with cryoprecipitate prepared within 5 days. Approximately 9 mls volume was kept in each bag. Six bags or units of plasma were used to illustrate the method. The Table sets out the total units of Factor VIII present in each of three Fractions obtained in sequence (Fraction 1 is the starting plasma, Fraction 2 of the cryoprecipitate pool and Fraction 3 the cold-insoluble precipitate); the Factor VIII recovery level in comparison to the starting level; the protein recovery level; the specific activity of the Factor VIII units per milligram of protein; the purity of the Factor VIII; the volumes of each of the fractions; and the volume level.

TABLE 1

|  | Fraction 1 | Fraction 2 | Fraction 3 |
|---|---|---|---|
| Total Factor VIII (Units) | 1633 | 1325 | 1017 |
| F. VIII Recovery (%) | 100 | 81 | 62 |
| Total Protein (mg) | 131,520 | 4770 | 1220 |
| Protein Recovery (%) | 100 | 3.6 | 0.9 |
| Specific Activity (U/mg) | .0124 | .278 | .834 |
| Purity wrt Plasma | 1 x | 22.4 x | 67.25 x |
| Volume (ml) | 1527 | 53 | 11.3 |
| Volume Recovery (%) | 100 | 3.5 | 0.7 |

It will be seen that the cryoprecipitate pool and the cold-insoluble globulin precipitate fractions respectively provide 886 and 666 units per litre of Factor VIII or 81% and 62% recovery. This can be compared with the 17% recovery reported by Fekete et al in column 5, Example 1 of the US Reissue Patent discussed above; this recovery figure represents only 170 units per litre of starting plasma.

Table 2 gives an analysis of the total contents of Fractions 1, 2 and 3. Factor VIII was purified from the cryoprecipitate by cold-incubation at 0°C for 2 hours in the presence of CSH buffer. The 6 bags of CPD plasma to which heperin had been added were pooled after cryoprecipitate. The percentage component recovery figures were determined relative to the levels in the starting plasma. Protein was determined by Lowry assay with bovine serum albumin as standard. RID (Radial Immunodiffusion) plates for albumin or fibrinogen were used to determine the concentration of these components. Standard plasma containing known amounts of albumin and fibrinogen were provided by Behring Diagnostics.

TABLE 2

|  | Fraction 1 | Fraction 2 | Fraction 3 |
|---|---|---|---|
| Total Protein (mg) | 131,520 | 4770 | 1220 |
| Protein Recovery (%) | 100 | 3.6 | 0.9 |
| Total Fibrinogen (mg) | 4404 | 424 | 384 |
| Fibrinogen Recovery (%) | 100 | 9.6 | 8.7 |
| Total Albumin (mg) | 65,906.4 | 1908 | 163 |
| Albumin Recovery (%) | 100 | 2.9 | .25 |
| Antibody Titre | 1:32 | 1:32 | 1:16 |
| Volume (ml) | 1527 | 53 | 11.3 |
| Volume Recovery (%) | 100 | 3.5 | .74 |

Application of the cryoprecipitate-cold insoluble globulin procedure to citrate plasma obtained by plasmapheresis using Haemonetics Model 50 machine with the plasma made to 1 unit of sodium heparin per ml of plasma also produces a Factor VIII concentrate of considerable potency. This technique results in the recovery of 81—83% of the initial plasma Factor VIII:C activity in the cryoprecipitate and an overall recovery in the final cold-insoluble globulin precipitate of 56—62% of the initial plasma Factor VIII activity. The specific activity of the final product is 0.96—0.99 units/mg of protein with a 99—160 fold increase in purity compared to plasma. As indicated in Tables 4 and 5, the data indicate that, when using plasmapheresis plasma, it is possible to increase the recovery to produce a concentrate with a specific activity which qualifies the compound as high purity (greater than 1 unit/mg).

TABLE 3

Recovery of F.VIII:C/ml From Citrate and 1 Unit Heparin Per ml Pheresis
Plasma by the Cold-Insoluble Cryoprecipitate Technique

| Sample | % F.VIII: C/ml | Volume | Total Units F.VIII:C | % Recovery Over Plasma | Unit/Liter Starting Plasma |
|---|---|---|---|---|---|
| Tubes: | | | | | |
| CPD Plasma | 60%/ml | — | — | — | — |
| Citrate Plasma | 60%/ml | — | — | — | — |
| Pheresis: | | | | | |
| Citrate Plasma and Heparin (1 U/ml) | 50%/ml | 2032 mls | 1219U | 100% | 600U/L |
| Cryoprecipitate Pool | 1120%/ml | 85 mls | 1015U | 83% | 500U/L |
| Cold-Insoluble Cryoprecipitate (Washed) | 2520%/ml | 28.1 mls | 755U | 62% | 372U/L |

5

Four volunteers (3 O's 1A) were plasmapheresed on a model 50 Haemonetics pheresis machine with 1 part 4 g% sodium citrate per 14 parts blood. They were also collected into CPD or citrate tubes as usual.

TABLE 4
Specific Activity of Citrate and Heparin Pheresis Plasma Fractions

| Fraction | Total F.VIII:C | Total Protein (mg) | Specific Activity | Purity Over Plasma |
|---|---|---|---|---|
| Plasma (Citrate + 1 Unit Heparin/ml) | 1219U | 195,072 | 0.006U/mg | — |
| Cryoprecipitate Pool | 1015U | 5,032 | 0.201U/mg | 33.5x |
| Cold-Insoluble Cryoprecipitate | 755.3U | 786.8 | 0.96U/mg | 160x |

It is believed that the method of the invention will be of utility in blood donor centres, and possibly also in the larger scale recovery of Factor VIII.

**Claims**

1. A method of obtaining Factor VIII comprising freezing plasma that is freshly obtained blood plasma collected into a calcium chelating agent or that is obtained by plasmapheresis using a calcium chelating agent, resolubilising the frozen plasma, resolubilising a cryoprecipitate obtained from the resolubilised plasma, adding a citrate saline buffer to the resolubilised cryoprecipitate and separating Factor VIII from the resolubilised cryoprecipitate, characterised in that heparin is incorporated with the plasma before it is frozen, the buffer includes heparin, and the separation of the Factor VIII from the resolubilised cryoprecipitate is effected by incubating the resolubilised cryoprecipitate in the presence of cold insoluble globulin that is precipitatable by heparin and thereby obtaining a precipitate rich in Factor VIII and also including cold insoluble globulin, and isolating Factor VIII from this precipitate.

2. A method according to claim 1 in which the plasma that is to be frozen is obtained by adding heparin to freshly obtained plasma that has been collected into citrate phosphate dextrose anticoagulant.

3. A method according to any preceding claim characterised in that the incubation of the buffered and resolubilised cryoprecipitate is conducted at from 0 to 10°C for at least one hour.

4. A method according to any preceding claim characterised in that the resolubilisation of the cryoprecipitate is effected at about 37°C for 2 to 5 minutes.

5. A method according to any preceding claim in which the resolubilisation of the frozen plasma is effected at 0 to 10°C for 30 to 120 minutes.

6. A method according to any preceding claim characterised in that the plasma that is to be frozen is freshly obtained blood plasma that has been collected into a calcium chelating anticoagulat and to which heparin is added or is blood plasma obtained by plasma pheresis using heparin and a calcium chelating anticoagulant.

7. A method according to any preceding claim in which the calcium chelating anticoagulant is a citrate anticoagulant, ethylenediaminetetraacetic acid or an oxalate.

8. A method according to any preceding claim in which the calcium chelating anticoagulant is an acid citrate dextrose or a citratephosphate dextrose.

9. A method according to any preceding claim characterised in that the cryoprecipitate is resolubilised in a bag and the amount of citrate saline heparin buffer added to each bag is from 2 to 10 ml.

10. A method according to any preceding claim characterised in that the isolation of the Factor VIII from the precipitate obtained by incubation is effected by washing the precipitate at from 0 to 10°C with citrate saline heparin buffer, draining the precipitate and resolubilising the precipitate in the presence of further citrate saline heparin buffer.

**Revendications**

1. Un procédé d'obtention du facteur VIII comprenant la congélation de plasma qui est du plasma sanguin fraîchement obtenu recueilli sur un agent chélatant le calcium ou qui est obtenu par plasmaphérèse avec emploi d'un agent chélatant le calcium, la resolubilisation du plasma congelé, la resolubilisation d'un cryoprécipité obtenu à partir du plasma resolubilisé, l'addition d'un tampon salé citraté au cryoprécipité resolubilisé et la séparation du facteur VIII du cryoprécipité resolubilisé, caractérisé en ce que de l'héparine est incorporée au plasma avant qu'il soit congelé, le tampon comprend de l'héparine et la séparation du facteur VIII du cryoprécipité resolubilisé est effectuée par incubation du cryoprécipité resolubilisé en présence de globuline insoluble à froid qui est précipitable par l'héparine pour obtenir ainsi un précipité riche en facteur VIII et comprenant également la globuline insoluble à froid et l'isolement du facteur VIII à partir de ce précipité.

2. Un procédé selon la revendication 1, dans lequel le plasma qui est congelé est obtenu par addition d'héparine à du plasma fraîchement obtenu qu'on a recueilli sur de l'anticoagulant citrate-phosphate-dextrose.

3. Un procédé selon l'une quelconque des revendications précédentes, caractérisé en ce que l'incubation du cryoprécipité tamponné et resolubilisé est effectuée entre 0 et 10°C pendant au moins 1 heure.

4. Un procédé selon l'une quelconque des revendications précédentes, caractérisé en ce que la resolubilisation du cryoprécipité est effectuée à environ 37°C pendant 2 à 5 minutes.

5. Un procédé selon l'une quelconque des revendications précédentes, dans lequel la resolubilisation du plasma congelé est effectuée entre 0 et 10°C pendant 30 à 120 minutes.

6. Un procédé selon l'une quelconque des revendications précédentes, caractérisé en ce que le plasma qui doit être congelé est du plasma sanguin fraîchement obtenu qui a été recueilli sur un anticoagulant chélatant le calcium et auquel de l'héparine est ajoutée, ou est un plasma sanguin obtenu par plasmaphérèse avec emploi d'héparine et d'un anticoagulant chélatant le calcium.

7. Un procédé selon l'une quelconque des revendications précédentes, dans lequel l'anticoagulant chélatant le calcium est un anticoagulant de type citrate, l'acide éthylène-diaminetétraacétique ou un oxalate.

8. Un procédé selon l'une quelconque des revendications précédentes, dans lequel l'anticoagulant chélatant le calcium est un anticoagulant acide-citrate-dextrose ou un anticoagulant citrate-phosphate-dextrose.

9. Un procédé selon l'une quelconque des revendications précédentes, caractérisé en ce que le cryoprécipité est resolubilisé dans un sac et la quantité de tampon salé citraté héparine ajoutée à chaque sac est de 2 à 10 ml.

10. Un procédé selon l'une quelconque des revendications précédentes, caractérisé en ce que l'isolement du facteur VIII à partir du précipité obtenu par incubation est effectué par lavage du précipité entre 0 et 10°C avec du tampon salé citraté hépariné, essorage du précipité et résolubilisation du précipité en présence d'une quantité additionnelle de tampon salé citraté hépariné.

**Patentansprüche**

1. Verfahren zur Gewinnung von Faktor VIII, welches umfaßt: das Einfrieren von Plasma, welches frisch aus Blutplasma erhalten und in Kalzium-Chelierungsmittel gesammelt wurde, oder welches durch Plasmaphorese unter Verwendung eines Kalzium-Chelierungsmittels erhalten wurde, das Wiederlöslichmachen des gefrorenen Plasmas, das Wiederlöslichmachen eines Cryopräzipitats, das man aus dem weider-löslich-gemachten Plasma erhielt, die Zugabe eines Zitrat-Salz-Puffers zu dem weider-löslich-gemachten Cryopräzipitat und das Abtrennen des Faktor VIII von dem wieder-löslich-gemachten Cryopräzipitat, dadurch gekennzeichnet, daß in das Plasma vor dem Einfrieren Heparin eingebracht wird, der Puffer Heparin umfaßt, und die Abtrennung des Faktor VIII von dem wieder-löslich-gemachten Cryopräzipitat durch Inkubation des wieder-löslich-gemachten Cryopräzipitats in Anwesenheit eines kalt-unlöslichen Globulins erfolt, das durch Heparin ausgefällt werden kann, und wobei ein Präzipitat erhalten wird, das reich an Faktor VIII ist und auch kalt-unlösliches Globulin umfaßt, und Abtrennen des Faktor VIII von diesem Präzipitat.

2. Verfahren nach Anspruch 1, wonach das einzufrierende Plasma erhalten wird, indem Heparin zu dem frisch gewonnenen Plasma zugegeben wird, welches in Zitrat-Phosphat-Dextrose-Antikoagulationsmittel gesammelt worden war.

3. Verfahren nach einem der vorausgehenden Ansprüche, dadurch gekennzeichnet, daß die Inkubation des gepufferten und wieder-löslich-gemachten Cryopräzipitats für die Dauer von mindestens 1 Stunde bei 0 bis 10°C durchgeführt wird.

4. Verfahren nach einem der vorausgehenden Ansprüche, dadurch gekennzeichnet, daß die Wiederlöslichmachung des Cryopräzipitats bei etwa 37°C für die Dauer von 2 bis 5 Minuten erfolgt.

5. Verfahren nach einem der vorausgehenden Ansprüche, wonach das Wiederlöslichmachen des gefrorenen Plasmas bei 0 bis 10°C für die Dauer von 30 bis 120 Minuten erfolgt.

6. Verfahren nach einem der vorausgehenden Ansprüche, dadurch gekennzeichnet, daß das einzufrierende Plasma frisch aus Blutplasma erhalten wurde, welches in Kalzium-Chelierungs-Antikoagulierungsmittel gesammelt wurde und zu welchem Heparin zugegeben wurde, oder Blutplasma ist, das durch Plasmaphorese unter Verwendung von Heparin und einem Kalzium-Chelierungs-Antikoagulierungsmittel erhalten wurde.

7. Verfahren nach einem der vorausgehenden Ansprüche, worin das Kalzium-Chelierungs-Antikoagulierungsmittel ein Zitrat-Antikoagulierungsmittel, Ethylendiamintetraessigsäure oder ein Oxalat ist.

8. Verfahren nach einem der vorausgehenden Ansprüche, worin das Kalzium-Chelierungs-Antikoagulierungsmittel saures Zitrat-Dextrose oder Zitrat-Phosphat-Dextrose ist.

9. Verfahren nach einem der vorausgehenden Ansprüche, dadurch gekennzeichnet, daß das Cryopräzipitat in einem Beutel wieder flüssig gemacht wird und die zugegebene Menge an Zitrat-Salz-Heparin-Pfuffer zu jedem Beutel von 2 bis 10 ccm beträgt.

10. Verfahren nach einem der vorausgehenden Ansprüche, dadurch gekennzeichnet, daß die Isolation des Faktor VIII von dem Präzipitat, das durch Inkubation erhalten wurde, durch Waschen des Präzipitats bei Temperaturen von 0 bis 10°C mit Zitrat-Salz-Heparin-Puffer durchgeführt wird, das Präzipitat drainiert und in Anwesenheit weiterer Zitrat-Salz-Heparin-Puffers wieder löslich gemacht wird.

FRESH CPD PLASMA¹ MADE
TO 1 UNIT HEPARIN PER ML.*

TOTAL F VIII: 1633 UNITS
TOTAL FIBRINOGEN: 4,404 MG
TOTAL PROTEIN: 131,520 MG

FREEZE:-80°C
THAW: 4°C x 75 MIN.
SPIN: 7000 x g
        7 MIN. AT 0°C
DRAIN

SUPERNATANT OF
CRYOPRECIPITATE

CRYOPRECIPITATE²

RESOLUBILIZE: 37°C
2-5 MIN.
ADD: 2.5 ML CSH BUFFER
INCUBATE: 0°C x 2 HR.
SPIN: 7000 xg
        7 MINS. AT 0°C
DRAIN

² TOTAL F VIII: 1325 UNITS
  TOTAL FIBRINOGEN: 424 MG.
  TOTAL PROTEIN: 4770 MG.

COLD SOLUBLE
SUPERNATANT

COLD INSOLUBLE
PRECIPITATE

WASH: 0°C
ADD: 10 ML COLD CSH BUFFER
DRAIN

COLD INSOLUBLE
PRECIPITATE

RESOLUBILIZE: 37°C, 5 MIN.
ADD: 2 ML CSH BUFFER
SPIN: 3700x g , 6 MIN.

FACTOR VIII³
RICH SOLUTION

DEBRIS

³TOTAL F. VIII: 1017 UNITS
  TOTAL FIBRINOGEN: 384 MG
  TOTAL: 1220 MG

* REPRESENTS A POOL OF
  6 UNITS OF PLASMA

1